(19) Europäisches Patentamt
European Patent Office
Office européen des brevets

(11) Veröffentlichungsnummer : **0 078 004**
**B1**

# (12) EUROPÄISCHE PATENTSCHRIFT

(45) Veröffentlichungstag der Patentschrift : **30.01.85**

(51) Int. Cl.⁴ : **C 25 B 3/02**, C 07 D307/32

(21) Anmeldenummer : **82109666.6**

(22) Anmeldetag : **20.10.82**

## (54) Elektrochemisches Verfahren zur Herstellung von 2,5-Dialkoxy-2,5-dihydrofuranen.

(30) Priorität : **28.10.81 DE 3142626**

(43) Veröffentlichungstag der Anmeldung :
**04.05.83 Patentblatt 83/18**

(45) Bekanntmachung des Hinweises auf die Patenterteilung : **30.01.85 Patentblatt 85/05**

(84) Benannte Vertragsstaaten :
**BE CH DE FR GB IT LI NL**

(56) Entgegenhaltungen :
**DE-B- 2 710 420**

(73) Patentinhaber : **BASF Aktiengesellschaft**
**Carl-Bosch-Strasse 38**
**D-6700 Ludwigshafen (DE)**

(72) Erfinder : **Degner, Dieter, Dr.**
**Kurpfalzstrasse 8**
**D-6701 Dannstadt-Schauernheim (DE)**
Erfinder : **Hannebaum, Heinz**
**Ostring 56**
**D-6700 Ludwigshafen (DE)**

**EP 0 078 004 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues elektrochemisches Verfahren zur Herstellung von 2,5-Dialkoxy-2,5-dihydrofuranen aus 2,5-Dihydrofuran oder Monoalkoxydihydrofuran und Alkanolen.

2,5-Dialkoxy-2,5-dihydrofurane lassen sich nach einem in Organic Syntheses, Vol. *40,* S. 29 [1960] beschriebenen Verfahren durch Umsetzung von Furan mit Brom in alkanolischer Lösung bei Temperaturen unter – 20 °C herstellen. Bei dieser Synthese ist es von Nachteil, daß sehr erhebliche Mengen an Brom und Hilfsbasen benötigt werden, die zu erheblichen Abwasserbelastungen führen. Technisch wenig attraktiv sind auch die erforderlichen tiefen Temperaturen. Diese Nachteile lassen sich vermeiden, wenn man die 2,5-Dialkoxy-2,5-dihydrofurane durch elektrochemische Oxidation von Furan herstellt. Ein technisch gut geeignetes Verfahren wird in der DE-AS 27 10 420 beschrieben. Aber auch hier wird das technisch nur unter erheblichem Sicherheitsaufwand handhabbare Furan als Ausgangsmaterial benötigt.

Es wurde nun gefunden, daß man 2,5-Dialkoxy-2,5-dihydrofurane der allgemeinen Formel

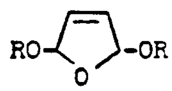

(I)

in der R ein Alkylrest mit 1 bis 4 C-Atomen bedeutet, auf besonders einfache Weise dadurch herstellen kann, daß man 2,5-Dihydrofuran oder Monoalkoxydihydrofuran der allgemeinen Formel

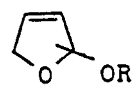

in Gegenwart eines Alkanols der Formel ROH elektrochemisch oxidiert.

2,5-Dihydrofuran ist sehr leicht aus Butendiol-1,4 durch Wasserabspaltung herstellbar und läßt sich technisch wesentlich einfacher als Furan handhaben. Daß man nach dem erfindungsgemäßen Verfahren aus dem 2,5-Dihydrofuran die 2,5-Dialkoxy-2,5-dihydrofurane so glatt herstellen kann, ist überraschend, da aus J. Am. Chem. Soc. 94, (1972), 7 892 bekannt ist, daß Olefine bei elektrochemischen Oxidationen häufig nur sehr schlechte Material- und Stromausbeuten ergeben. Insbesondere die regioselektive Einführung zweier Alkoxygruppen bei dem erfindungsgemäßen Verfahren ist sehr überraschend.

Das erfindungsgemäße Verfahren läßt sich am Beispiel der Synthese von 2,5-Dimethoxy-2,5-dihydrofuran durch nachfolgendes Formelschema näher verdeutlichen :

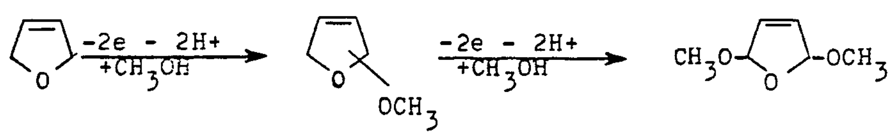


Bei der Synthese wird Monomethoxydihydrofuran als Zwischenstufe durchlaufen. Man kann das erfindungsgemäße Verfahren also auch so durchführen, daß man anstelle von 2,5-Dihydrofuran ein Monoalkoxydihydrofuran der allgemeinen Formel

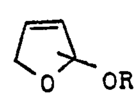

(II)

in der R die oben genannte Bedeutung hat, verwendet.

Das erfindungsgemäße Verfahren benötigt keine speziellen Elektrolysezellen, es kann in den technisch üblichen geteilten und ungeteilten Zellen durchgeführt werden. Die Elektrooxidation von 2,5-Dihydrofuran wird vorzugsweise in einem Elektrolyten durchgeführt, der aus 2,5-Dihydrofuran, einem Alkanol und einem Leitsalz besteht. Als Alkanol verwendet man z. B. Methanol, Ethanol, n-Propanol und Isopropanol.

Bevorzugtes Alkanol ist Methanol. Als Leitsalze werden bei dem erfindungsgemäßen Verfahren solche eingesetzt, die sich in dem 2,5-Dihydrofuran-Alkanol-Gemisch lösen und eine Leitfähigkeit der Lösung ermöglichen. Solche Leitsalze sind z. B. Halogenide, wie KF und NaBr, Alkoholate wie $NaOCH_3$, Salze von Alkansäuren, wie Kaliumacetat, Sulfonate, wie Kaliumbenzolsulfonat und Tetraethylammonium-p-toluolsulfonat, Alkylsulfate, wie Tetramethylammoniummethosulfat, Tetrafluoroborate, wie $LiBF_4$ und Hexafluorophosphate, wie $KPF_6$. Es können auch Gemische dieser Leitsalze oder auch Basen, wie

KOH als Leitsalz eingesetzt werden. Bevorzugt werden bei dem erfindungsgemäßen Verfahren Halogenide, Sulfonate oder Alkoholate als Leitsalze eingesetzt. Besonders bevorzugt wird Kaliumbenzolsulfonat verwendet.

Die Elektrolytzusammensetzung kann in weiten Grenzen gewählt werden. Der Elektrolyt setzt sich beispielsweise wie folgt zusammen :

5 bis 50 Gew.-% 2,5-Dihydrofuran
1 bis 10 Gew.-% Leitsalz
40 bis 94 Gew.-% Alkanol

Als Anodenmaterialien werden solche Werkstoffe eingesetzt, die unter den Elektrolysebedingungen stabil sind. Beispiele hierfür sind Edelmetalle, wie Platin, Oxide, wie Rutheniumdioxid auf Titan, Graphit und glasartiger Kohlenstoff. Bevorzugt werden Platin- und Graphitanoden eingesetzt. Als Kathodenmaterialien können beispielsweise Metalle, wie Eisen, Metallegierungen, wie Stahl, Edelmetalle, wie Platin oder Graphit eingesetzt werden.

Die Stromdichten können ebenfalls in weiten Grenzen gewählt werden ; sie betragen beispielsweise 1 bis 30 A/dm$^2$. Bevorzugt werden Stromdichten zwischen 2 und 15 A/dm$^2$ angewandt. Die Elektrolysetemperatur ist durch die Siedetemperatur des 2,5-Dihydrofurans begrenzt, sie liegt daher zweckmäßigerweise unter 60 °C.

Der Umsatz von 2,5-Dihydrofuran ist in weiten Grenzen wählbar ; bevorzugt wird 2,5-Dihydrofuran zu über 50 % umgesetzt. Unumgesetztes 2,5-Dihydrofuran kann zusammen mit eventuell noch vorhandenem Monomethoxydihydrofuran zur Elektrolyse zurückgeführt werden.

Die Aufarbeitung der Elektrolyseausträge erfolgt nach an sich bekannten Methoden. So wird der überschüssige Alkohol zusammen mit eventuell noch vorhandenem 2,5-Dihydrofuran bei Normaldruck abdestilliert. Das anfallende Destillat wird zur Elektrolyse zurückgeführt. Das im Destillationssumpf anfallende Leitsalz wird vom Roh-Dialkoxydihydrofuran z. B. durch Filtration abgetrennt und kann ebenfalls zur Elektrolyse zurückgeführt werden. Die 2,5-Dialkoxy-2,5-dihydrofurane können z. B. durch Rektifikation weiter gereinigt werden. Das erfindungsgemäße Verfahren kann sowohl diskontinuierlich als auch kontinuierlich durchgeführt werden.

Beispiele 1 bis 4

Als Elektrolysezelle wurde in allen Beispielen eine Plattenstapelzelle (ungeteilte Zelle) eingesetzt. Der Elektrodenstapel bestand aus 6 bis 11 Elektroden, die Elektrodenabstände betrugen 0,5 mm. Der Elektrolyt wurde während der Elektrolyse mit 200 l/h über einen Wärmetauscher gepumpt. Die Elektrolyse wurde diskontinuierlich durchgeführt. Die Elektrolyseausträge wurden destillativ aufgearbeitet. Zunächst wurde Methanol zusammen mit eventuell noch vorhandenem 2,5-Dihydrofuran bei Normaldruck abdestilliert, das Leitsalz danach vom Roh-2,5-Dimethoxy-2,5-dihydrofuran abdestilliert und das Roh-Dimethoxy-dihydrofuran bei 80 bis 100 °C und 120 bis 150 mbar reindestilliert.

Die Gehaltsbestimmungen wurden gaschromatographisch durchgeführt. Einzelheiten der Versuche sowie der Versuchsergebnisse sind in nachfolgender Tabelle zusammengestellt.

(Siehe Tabelle Seite 4 f.)

## Elektrosynthese von 2,5-Dimethoxy-2,5-dihydrofuran

| Beispiel | Elektrodenmaterial | Elektrolyt (g) | Stromdichte ($A/dm^2$) | Elektrolyse mit Q (F/Mol DHF) | T ($^{o}C$) | DHF (g) | MDF (g) | DMD (g) | Umsatz DHF (%) | MA (%) | SA (%) |
|---|---|---|---|---|---|---|---|---|---|---|---|
| 1 | A: Graphit<br>K: Graphit | 280 DHF<br>21.4 $KSO_3C_6H_5$<br>2291 $CH_3OH$ | 4 | 4 | 25-27 | 7,7 | - | 342,3 | 97,3 | 67,7 | 65,8 |
| 2 | A: Graphit<br>K: Graphit | 280 DHF<br>25 $NaOCH_3$<br>2251 $CH_3OH$ | 2-4 | 4 | 25-27 | 15,6 | 42,0 | 230,9 | 94,4 | 52,9 | 49,7 |
| 3 | A: Graphit<br>K: Graphit | 280 DHF<br>25 KOAc<br>2251 $CH_3OH$ | 2-4 | 4 | 22-29 | 28,7 | 44,4 | 177,1 | 89,8 | 43,3 | 39,6 |
| 4 | A: Graphit<br>K: Graphit | 280 DHF<br>25 KF<br>2370 $CH_3OH$ | 4 | 3 | 25-27 | 122,1 | 71,6 | 156,6 | 56,4 | 78,2 | 52,1 |

T : Elektrolysetemperatur

DHF : 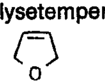

MDF : 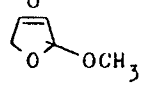

DMD : 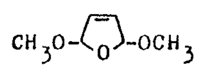

A : Anode
K : Kathode
MA : Materialausbeute
SA : Stromausbeute

**Ansprüche**

1. Verfahren zur Herstellung von 2,5-Dialkoxy-2,5-dihydrofuranen der allgemeinen Formel

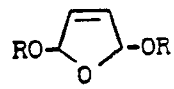

(I)

in der R ein Alkylrest mit 1 bis 4 C-Atomen bedeutet, dadurch gekennzeichnet, daß man 2,5-Dihydrofuran oder Monoalkoxydihydrofuran der allgemeinen Formel

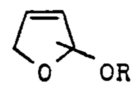

in Gegenwart eines Alkanols der Formel ROH elektrochemisch oxidiert.

2. Verfahren nach Anspruch 1, dadurch gekennzeichnet, daß man als Alkanol Methanol verwendet.

3. Verfahren nach den Ansprüchen 1 und 2, dadurch gekennzeichnet, daß man die elektrochemische Oxidation an Graphit- oder Platinanoden durchführt.

4. Verfahren nach den Ansprüchen 1 und 2, dadurch gekennzeichnet, daß man Alkoholate, Sulfonate oder Halogenide als Leitsalze einsetzt.

**Claims**

1. A process for the preparation of a 2,5-dialkoxy-2,5-dihydrofuran of the general formula

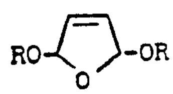

(I)

where R is alkyl of 1 to 4 carbon atoms, wherein 2,5-dihydrofuran or monoalkoxydihydrofuran of the general formula

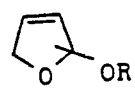

is oxidized electrochemically in the presence of an alkanol of the formula ROH.

2. A process as claimed in claim 1, wherein the alkanol used is methanol.

3. A process as claimed in claims 1 and 2, wherein the electrochemical oxidation is carried out on a graphite or platinum anode.

4. A process as claimed in claims 1 and 2, wherein the conducting salt used is an alcoholate, a sulfonate or a halide.

**Revendications**

1. Procédé de préparation de dialcoxy-2,5 dihydro-2,5 furannes de la formule générale

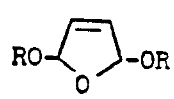

(I)

dans laquelle R désigne un radical alcoyle en $C_1$ à $C_4$, caractérisé en ce que l'on soumet le dihydro-2,5 furanne ou un mono-alcoxy-dihydro-furanne de la formule générale

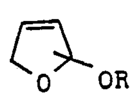

en présence d'un alcanol de la formule ROH à une oxydation électrochimique.

2. Procédé suivant la revendication 1, caractérisé en ce que l'alcanol utilisé est l'alcool méthylique.

3. Procédé suivant les revendications 1 et 2, caractérisé en ce que l'oxydation électrochimique est réalisée sur des anodes en graphite ou en platine.

4. Procédé suivant les revendications 1 et 2, caractérisé en ce que l'on emploie des alcoolates, des sulfonates ou des halogénures comme sels conducteurs.